(19) Europäisches Patentamt
European Patent Office
Office européen des brevets

(11) Publication number : **0 458 600 B1**

# (12) EUROPEAN PATENT SPECIFICATION

(45) Date of publication of patent specification : **02.03.94 Bulletin 94/09**

(51) Int. Cl.$^5$ : **A61K 7/00,** A61K 7/50, A61K 7/06, A61K 7/48, C11D 1/66

(21) Application number : **91304604.1**

(22) Date of filing : **21.05.91**

(54) **Cosmetic emulsion.**

(30) Priority : **24.05.90 GB 9011696**

(43) Date of publication of application : **27.11.91 Bulletin 91/48**

(45) Publication of the grant of the patent : **02.03.94 Bulletin 94/09**

(84) Designated Contracting States : **AT BE CH DE DK ES FR GB GR IT LI NL SE**

(56) References cited :
**DE-A- 2 339 149**
**FR-A- 2 452 314**
**CHEMICAL ABSTRACTS, vol. 82, no. 18, 5th May 1975, page 281, abstract no. 116002z, Columbus, Ohio, US; & JP-A 75 04 251 (DAIICHI KOGYO SEIYAKU CO., LTD) 17-01-1975**
**PATENT ABSTRACTS OF JAPAN, vol. 11, no. 18 (C-398)[2465], 17th January 1987; & JP-A-61 194 007 (SHISEIDO CO., LTD) 28-08-1986**

(73) Proprietor : **UNILEVER PLC**
**Unilever House Blackfriars P.O. Box 68**
**London EC4P 4BQ (GB)**
(84) **GB**
Proprietor : **UNILEVER N.V.**
**Weena 455**
**NL-3013 AL Rotterdam (NL)**
(84) **BE CH DE DK ES FR GR IT LI NL SE AT**

(72) Inventor : **Carter, Peter, Unilever Research Port Sunlight Laboratory, Quarry Road East Bebington, Wirral, Merseyside L63 3JW (GB)**
Inventor : **Hill, John Christopher, Unilever Research Port Sunlight Laboratory, Quarry Road East Bebington, Wirral, Merseyside L63 3JW (GB)**
Inventor : **Macauley, Ernest Weatherley, Unilever Research Port Sunlight Laboratory, Quarry Road East Bebington, Wirral, Merseyside L63 3JW (GB)**
Inventor : **Thom, David, Unilever Research Port Sunlight Laboratory, Quarry Road East Bebington, Wirral, Merseyside L63 3JW (GB)**

(74) Representative : **Linn, Samuel Jonathan et al**
**MEWBURN ELLIS 2 Cursitor Street**
**London EC4A 1BQ (GB)**

EP 0 458 600 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to an emulsion suitable for topical application to human skin and/or hair. More particularly, the invention is concerned with an emulsion having superior, smooth skin feel and lubricity properties when applied to skin, compared with conventional oil and water emulsions, yet which possesses less of the negative attributes, such as the persistent oily or greasy properties at the skin surface, which are more usually associated with such conventional emulsions when applied topically. These superior properties are derived from the use of special esters, instead of the oils that usually feature in conventional oil and water emulsions. Dependant upon the choice of these special esters the invention is furthermore concerned with an occlusive emulsion which not only possesses the superior properties referred to, but which also is capable of coating the skin with a water vapour retarding layer to reduce moisture loss from the skin over an extended period of time. Likewise, the occlusive emulsion can be employed to coat individual hair shafts to protect them from fracture or other damage, thus imparting to the hair a conditioning benefit.

### BACKGROUND TO THE INVENTION

A soft, supple and flexible skin has a marked cosmetic appeal and is an attribute of normal functioning stratum corneum.

As human skin ages with advancing years, the epidermis can become folded, ridged or furrowed to form wrinkles which signal the loss of youthful appearance and herald the transition to old age. This transition can occur prematurely, with young people, especially those who expose themselves to excessive doses of sunlight. Also, the outer layer of the epidermis, that is the stratum corneum, can become in appearance dry and flaky following exposure to cold, dry weather, or excessive contact with detergents or solvents which can result in a decrease in stratum corneum moisture, with the result that the skin loses its soft, supple and flexible characteristics.

Emollients such as fats, phospholipids and sterols have in the past been used in an attempt to soften wrinkled or dry skin, but it is apparent that these emollients are only partially effective as a remedy for skin in poor condition. This is likely in part to be due to the poor occlusive properties of the fats and lipid materials on the skin surface, where they are unable to reduce moisture loss from the underlying skin to any significant extent. Such poor occlusive properties may result from the presence of lipases, which occur naturally in the skin, and which tend to degrade fatty materials to produce free fatty acids and other breakdown products, so reducing any occlusive benefit that might otherwise have been achieved with intact lipids.

There accordingly exists a need for the provision of a stable lipid-like layer to be applied to skin in order to reduce moisture loss and to enable the skin to remain in a healthy, youthful condition, in the face of advancing years, which often is accompanied by the development of skin wrinkles and excessive sensitivity to adverse atmospheric or environmental conditions.

### PRIOR ART

In US patent 4,446,165 (The Procter & Gamble Company), there are disclosed cosmetic ointments comprising non-digestible fatty acid esters of a polyol compound, such as sucrose octaoleate, which include both a water-in-oil emulsion stabilising agent, and a destabilising surfactant, whose function is to invert the water-in-oil emulsion to form an oil-in-water emulsion when the emulsion is subjected to shear. This patent exemplifies shear as not only mastication in the mouth but also rubbing onto the skin. Clearly, the teaching of this reference is to a water-in-oil emulsion which readily breaks when rubbed onto the skin's surface and disperses and disappears therefrom. Certainly, there is no teaching that an occlusive layer could be formed following topical application of this emulsion.

JP-A-7504251 (Chemical Abstracts, vol. 82, no. 18, 5th May 1975, page 281, abstract no. 116002z, Daiichi Kogyo Seijaku Co., Ltd) also discloses creams and emulsion cosmetics containing, in addition to water and surfactant, a sucrose fatty acid ester such as sucrose pentastearate. Again, however, there is no teaching in this document of the formation of an occlusive layer on skin or hair following application of such a composition.

### SUMMARY OF INVENTION

Applicants, in their search for a product having skin feel and lubricity properties that are superior to those of conventional emulsions, and that preferably also provides an occlusive film on the skin following topical application thereon, have surprisingly found that an emulsion formed from an aqueous phase, and a non-aqueous phase comprising one or more special polyol fatty acid polyesters, can deliver just such properties, usually together with an occlusive film to the skin surface. An occlusivity test was devised to select those polyol fatty acid polyesters that are suitable for this purpose.

## DEFINITION OF THE INVENTION

Accordingly, the invention provides the use for the provision of an occlusive layer on skin or hair following topical application thereto of a cosmetic emulsion which comprises, in addition to water:

i. from 1 to 95% by weight of a polyol fatty acid polyester, the polyol having at least 4 free hydroxyl groups, at least 60% of which are then esterified with one or more fatty acids having from 8 to 22 carbon atoms;

ii. from 1 to 50% by weight of an emulsifier;

the emulsion having an aqueous phase which forms from 4 to 97% by volume, and a non-aqueous phase which forms from 3 to 96% by volume;

provided that when the emulsion is an oil-in-water emulsion, the emulsion is substantially free from an emulsifier having an average HLB value of <6.

## DISCLOSURE OF THE INVENTION

The emulsion used according to the invention can be in the form of an oil-in-water emulsion or a water-in-oil emulsion, dependent largely on the choice of emulsifier.

The emulsion used according to the invention comprises an aqueous phase which forms from 4 to 97% by volume and a non-aqueous phase which forms from 3 to 96% by volume, and is an occlusive emulsion, that is one which will provide an occlusive layer of polyol fatty acid polyester on skin or hair when topically applied thereto.

### The polyol fatty acid polyesters

The emulsion of the invention comprises one or more polyol fatty acid polyesters, which are fatty acid polyesters derived from any aliphatic or aromatic polyol which has at least 4 free hydroxyl groups, of which at least 60% of these free hydroxy groups are then esterified with one or more fatty acids having from 8 to 22 carbon atoms.

It is important that at least 60% of the free hydroxyl groups are esterified, as this renders the polyol fatty acid polyester resistant to cleavage by enzymes, particularly lipase.

The polyol from which the polyol fatty acid polyesters are derived are preferably chosen from sugar polyols, which comprise mono-, di- and polysaccharides.

Preferred examples of monosaccharide sugar polyols include:

Pentose sugar polyols such as D-ribose, D-arabinose, D-xylose, D-lyxose, D-ribulose and D-xylulose.

Hexose sugar polyols such as D-allose, D-altrose, D-glucose, D-mannose, D-gulose, D-idose, D-galactose, D-talose, D-fructose, D-sorbose and D-tagatose.

Heptose sugar polyols, such as D-mannoheptulose and D-sedoheptulose.

The polyol from which the polyol fatty acid polyester is derived can also be chosen from disaccharides such as maltose, lactose, cellobiose, sucrose, trehalose, gentiobiose, melibiose and primeverose.

The polyol from which the polyol fatty acid polyesters are derived can alternatively be chosen from: tri-saccharides, such as gentianose and raffinose.

The polyol from which the polyol fatty acid polyesters are derived can alternatively be chosen from: sugar alcohols such as D-mannitol, D-sorbitol, D-ribitol, D-erithritol, D-lactitol and D-xylitol.

The polyol from which the polyol fatty acid polyesters are derived can alternatively be chosen from derivatives of sugars such as -methyl glucoside and inositol.

The preferred sugar polyol is sucrose.

The fatty acids which are employed to form the polyol fatty acid polyesters of the invention can be individual free fatty acids having from 8 to 22 carbon atoms in the fatty acid molecule.

These fatty acids can be saturated or unsaturated, linear or branched chain fatty acids.

A preferred source of fatty acids for forming the polyol fatty acid polyesters are naturally occurring fats and oils which provide a source of a blend of fatty acids residues, whose choice can vary widely the physical and chemical properties of the polyol fatty acid polyesters obtained therefrom.

These naturally occurring fats and oils can be used as obtained from nature or following full or partial hydrogenation, interesterification, transesterification or fractionation.

Suitable natural sources of these fatty acid residues may be of animal, marine or vegetable origin, such

as coconut oil, palmkernel oil, palm oil, butter fat, soyabean oil, safflower oil, cotton seed oil, rapeseed oil, poppy seed oil, corn oil, sunflower oil, ground nut oil, fish oils and mixtures thereof. Preferred fatty acid sources are palm oils, partially hydrogenated palm oils, palmkernel oils, optionally partially hydrogenated soya bean oils and partially hydrogenated fish oils.

By employing a mixture of fatty acids, or one or more naturally occurring oils such as those exemplified above, when synthesising the polyol fatty acid polyester, it is possible to provide polyol fatty acid polyesters in which a mixture of ester groups is present on a single polyol molecule. In this way it is possible to vary the melting characteristics of the polyol fatty acid polyester so formed as desired.

The polyol which can be reacted with a source of fatty acids such as those herein described will, as has previously been stated, comprise at least 4 free hydroxy groups, any or all of which are available for esterification with the fatty acid moieties. Usually, at least 60% of these free hydroxy groups are esterified to provide the polyol fatty acid polyester which is to be employed in forming the emulsion of the invention. More preferably, 70% and ideally at least 80% of these free hydroxy groups are substituted with fatty acid ester groups.

Those polyol fatty acid polyesters which are useful in preparing the emulsion according to the invention which is occlusive, can be further characterised by possessing an Occlusivity Value of at least 50%, as measured by the Occlusivity Value Test. Preferred polyol fatty acid polyesters possess an Occlusivity Value of at least 60%, most preferably at least 70% and ideally of at least 80% as measured by this Test. Details of how this test is performed are given later in this specification.

Those polyol fatty acid polyesters that possess an Occlusivity Value of less than 50% are generally incapable, in the absence of polyol fatty acid polyesters having a higher Occlusivity Value, of yielding an emulsion according to the invention which is capable in use of producing an effective occlusive layer on skin. Such polyol fatty acid polyesters are nevertheless useful in preparing emulsions which possess other desirable attributes, such as the superior, smooth skin feel and lubricity properties referred to herein.

The amount of the polyol fatty acid polyester component to be incorporated in the emulsion of the invention is from 1 to 95%, preferably from 1 to 50% and most preferably from 1 to 20% by weight of the emulsion.

Emulsions containing less than 1% by weight of the polyol fatty acid polyester component are unlikely in use to be able to deliver to the skin or hair sufficient of the polyol fatty acid polyester to provide a satisfactory occlusive layer on the skin. Emulsions of the invention containing more than 95% by weight of the polyol fatty acid polyester are unlikely to deliver to the skin an occlusive layer of the polyol fatty acid polyester which is more effective than that derived from an emulsion containing 95% by weight of this component.

## THE EMULSIFIER

The emulsion of the invention also comprises one or more emulsifiers, the choice of which will normally determine whether a water-in-oil or and oil-in-water emulsion is formed.

When a water-in-oil emulsion is required, the chosen emulsifier or emulsifiers should have an average HLB value of from 1 to 6. When an oil-in-water emulsion is required, a chosen emulsifier or emulsifiers should have an average HLB value of >6.

Examples of suitable emulsifiers are set below in Table 1 in which the chemical name of the emulsifiers is given together with an example of a trade name as commercially available, together with the average HLB value.

Table 1

| Chemical Name of Emulsifier | Trade Name | HLB Value |
|---|---|---|
| Sorbitan trioleate | Arlacel 85 | 1.8 |
| Sorbitan tristearate | Span 65 | 2.1 |
| Glycerol monooleate | Aldo MD | 2.7 |
| Glycerol monostearate | Atmul 84S | 2.8 |
| Glycerol monolaurate | Aldo MC | 3.3 |
| Sorbitan sesquioleate | Arlacel 83 | 3.7 |
| Sorbitan monooleate | Span 80 | 4.3 |
| Sorbitan monostearate | Arlacel 60 | 4.7 |
| Poloxyethylene (2) stearyl ether | Brij 72 | 4.9 |
| Poloxyethylene sorbitol beeswax derivative | G-1702 | 5 |
| PEG 200 dilaurate | Emerest 2622 | 6.3 |
| Sorbitan monopalmitate | Arlacel 40 | 6.7 |
| Polyoxyethylene (3.5) nonyl phenol | Emulgen 903 | 7.8 |
| PEG 200 monostearate | Tegester PEG 200 MS | 8.5 |
| Sorbitan monolaurate | Arlacel 200 | 8.6 |
| PEG 400 dioleate | Tegester PEG 400-DO | 8.8 |
| Polyoxyethylene (5) monostearate | Ethofat 60-16 | 9.0 |
| Polyoxyethylene (4) sorbitan monostearate | Tween 61 | 9.6 |
| Polyoxyethylene (4) lauryl ether | Brij 30 | 9.7 |
| Polyoxyethylene (5) sorbitan monooleate | Tween 81 | 10.0 |

| | | |
|---|---|---|
| PEG 300 monooleate | Neutronyx 834 | 10.4 |
| Polyoxyethylene (20) sorbitan tristearate | Tween 65 | 10.5 |
| Polyoxyethylene (20) sorbitan trioleate | Tween 85 | 11.0 |
| Polyoxyethylene (8) monostearate | Myrj 45 | 11.1 |
| PEG 400 monooleate | Emerest 2646 | 11.7 |
| PEG 400 monostearate | Tegester PEG 400 | 11.9 |
| Polyoxyethylene 10 monooleate | Ethofat 0/20 | 12.2 |
| Polyoxyethylene (10) stearyl ether | Brij 76 | 12.4 |
| Polyoxyethylene (10) cetyl ether | Brij 56 | 12.9 |
| Polyoxyethylene (9.3) octyl phenol | Triton X-100 | 13.0 |
| Polyoxyethylene (4) sorbitan monolaurate | Tween 21 | 13.3 |
| PEG 600 monooleate | Emerest 2660 | 13.7 |
| PEG 1000 dilaurate | Kessco | 13.9 |
| Polyoxyethylene sorbitol lanolin derivative | G-1441 | 14.0 |
| Polyoxyethylene (12) lauryl ether | Ethosperse LA-12 | 14.4 |
| PEG 1500 dioleate | Pegosperse 1500 | 14.6 |
| Polyoxyethylene (14) laurate | Arosurf HFL-714 | 14.8 |
| Polyoxyethylene (20) sorbitan monostearate | Tween | 14.9 |
| Polyoxyethylene 20 sorbitan monooleate | Tween 80 | 15.0 |
| Polyoxyethylene (20) stearyl ether | Brij 78 | 15.3 |

| | | |
|---|---|---|
| Polyoxyethylene (20) sorbitan monopalmitate | Tween 40 | 15.6 |
| Polyoxyethylene (20) cetyl ether | Brij 58 | 15.7 |
| Polyoxyethylene (25) oxypropylene monostearate | G-2162 | 16.0 |
| Polyoxyethylene (20) sorbitol monolaurate | Tween 20 | 16.7 |
| Polyoxyethylene (23) lauryl ether | Brij 35 | 16.9 |
| Polyoxyethylene (50) monostearate | Myrj 53 | 17.9 |
| PEG 4000 monostearate | Pegosperse 4000 MS | 18.7 |

The foregoing list of emulsifiers is not intended to be limiting and merely exemplifies selected emulsifiers which are suitable for use in accordance with the invention.

It is to be understood that two or more emulsifiers can be employed if desired.

The amount of emulsifier or mixtures thereof, to be incorporated in the emulsion of the invention is from 1 to 50%, preferably from 2 to 20% and most preferably from 2 to 10% by weight of the emulsion.

Water

The emulsion of the invention also comprises water, usually from 4 to 97% by volume.

OTHER INGREDIENTS

Volatile siloxane

The emulsion of the invention can also optionally comprise a volatile polydimethylsiloxane such as polydimethylcyclosiloxane having a viscosity of less than $5mm^2s^{-1}$, examples of which are DOW CORNING 344 Fluid (tetramer) and DOW CORNING 345 Fluid (pentamer), and volatile hexamethyldisiloxane having a viscosity of not more than $0.65mm^2s^{-1}$, for example DOW CORNING 200 Fluid ($0.65mm^2s^{-1}$).

The preferred volatile siloxane when one is employed, is polydimethylcyclosiloxane (pentamer).

The amount of volatile siloxane, when present in the emulsion, will normally be up to 50%, preferably from 5 to 20% by weight of the emulsion.

Silicone Surfactant

The emulsion of the invention can also optionally comprise a high molecular weight silicone surfactant which can also act as an emulsifier, in place of or in addition to the emulsifier(s) already mentioned.

The silicone surfactant is a high molecular weight polymer of dimethyl polysiloxane with polyoxyethylene and/or polyoxypropylene side chains having a molecular weight of from 10,000 to 50,000 and having the structure:

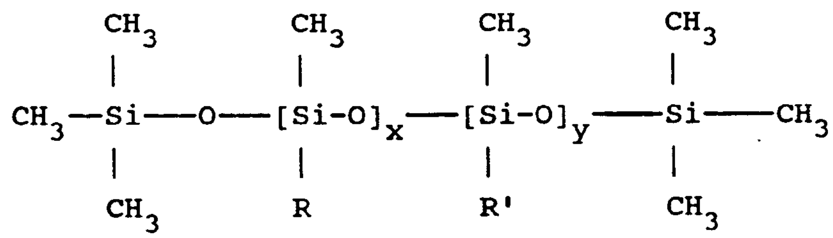

where

R is a $C_{1-18}$ alkyl group and

R' is a polyether group having the structure:

$$-CH_3H_5O(C_2H_4O)_a(CH_3C_2H_3O)_bR''$$

where R" is H or a $C_1$-$_{18}$ alkyl group,

a has a value of from 9 to 115,

b has a value of from 0 to 50,

x has a value of from 133 to 673,

y has a value of from 25 to 0.25.

Preferably, the dimethyl polysiloxane polymer is one in which:

R is methyl or lauryl

a has a value of from 10 to 114

b has a value of from 0 to 49

x has a value of from 388 to 402

y has a value of from 15 to 0.75

and R' has a molecular weight of from 1000 to 5000.

A particularly preferred dimethyl polysiloxane polymer is one in which:

a has the value 14

b has the value 13

x has the value 249

y has the value 1.25

The dimethyl polysiloxane polymer is conveniently provided as a dispersion in a volatile siloxane, the dispersion comprising, for example, from 1 to 20% by volume of the polymer and from 80 to 99% by volume of the volatile siloxane. Ideally, the dispersion consists of a 10% by volume of the polymer dispersed in the volatile siloxane.

Examples of the volatile siloxanes in which the polysiloxane polymer can be dispersed include those given above.

A particularly preferred silicone surfactant is cyclomethicone and dimethicone copolyol, such as DC 3225C Formulation Aid available from DOW CORNING. Others include laurylmethicone copolyol, such as DC Q2-5200, and the dimethicone copolyols DC Q2-5220, DC 190 and DC 193, all available from Dow Corning.

The amount of silicone surfactant, when present in the emulsion will normally be up to 25%, preferably from 0.5 to 15% by weight of the emulsion.

Non-volatile siloxane

The emulsion can also, optionally, comprise a non-volatile siloxane such as a polydimethylsiloxane having a viscosity in excess of $5mm^2s^{-1}$, for example, from 50 to $1000mm^2s^{-1}$, for example DOW CORNING 200 Fluids (standard viscosities 50-$1000mm^2s^{-1}$).

Inorganic electrolyte

The emulsion of the invention can also optionally comprise an inorganic electrolyte which can serve to improve the stability of the emulsion, particularly when subjected during storage to extremes of temperature.

Examples of inorganic electrolytes include salts, such as alkali metal and ammonium halides, sulphates, nitrates, carbonates and bicarbonates in either anhydrous or hydrated form.

Particularly preferred salts include sodium chloride, potassium chloride and ammonium chloride.

The amount of inorganic electrolyte, when present in the emulsion will normally be up to 10%, preferably from 0.2 to 5% by weight of the emulsion.

Alkane diol

The emulsion of the invention can also optionally comprise an alkane diol, or a mixture thereof, which can serve further to improve and prolong the stability of the emulsion, particularly when a very long period of storage, for example of at least 12 months or even up to 3 years, is anticipated.

The preferred alkane diols for this purpose are those having from 2 to 10 carbon atoms in the molecule. Examples of particularly preferred alkane diols are:

ethane diol
propane-1,2-diol
propane-1,3-diol
butane-1,3-diol
butane-1,4-diol
butane-2,3-diol
pentane-1,5-diol
hexane-1,6-diol
octane-1,8-diol,and
decane-1,10-diol

An especially preferred alkane diol is butane-1,3-diol.

The amount of alkane diol, when present in the emulsion will normally be up to 30%, most preferably from 1 to 25% by weight of the emulsion.

Cosmetically Acceptable Vehicle

The emulsion of the invention can optionally comprise a cosmetically acceptable vehicle, in addition to water, to act as a dilutant, dispersant or carrier for other materials present in the emulsion, so as to facilitate their distribution when the emulsion is applied to the skin and/or hair.

Vehicles other than water can include liquid or solid emollients, solvents, humectants, thickeners and powders. Examples of each of these types of vehicle, which can be used singly or as mixtures of one or more vehicles, are as follows:

Emollients, such as stearyl alcohol, glyceryl monoricinoleate, glyceryl monostearate, mink oil, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, eicosanyl alcohol, behenyl alcohol, cetyl palmitate, silicone oils such as dimethylpolysiloxane, di-n-butyl sebacate, isopropyl myristate, isopropyl palmitate, isopropyl stearate, butyl stearate, triethylene glycol, lanolin, cocoa butter, corn oil, cotton seed oil, tallow, lard, olive oil, palm kernel oil, rapeseed oil, safflower seed oil, evening primrose oil, soybean oil, sunflower seed oil, avocado oil, sesame seed oil, coconut oil, arachis oil, castor oil, acetylated lanolin alcohols, petroleum jelly, mineral oil, butyl myristate, isostearic acid, palmitic acid, isopropyl linoleate, lauryl lactate, myristyl lactate, decyl oleate, myristyl myristate;

Propellants, such as propane, butane, isobutane, dimethyl ether, carbon dioxide, nitrous oxide;

Powders, such as chalk, talc, fullers earth, kaolin, starch, gums, colloidal silica sodium polyacrylate, tetra alkyl and/or trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose, ethylene glycol monostearate, titanium dioxide, titanium dioxide-coated mica.

The cosmetically acceptable vehicle, when present, will usually form up to 95%, preferably from 10 to 90% by weight of the emulsion, and can, in the absence of other cosmetic adjuncts, form the balance of the emulsion.

Cosmetic Adjuncts

Examples of conventional adjuncts which can optionally be employed include preservatives, such as parahydroxy benzoate esters; antioxidants, such butyl hydroxy toluene; humectants, such as glycerol, 2-pyrrolidone-5-carboxylate, dibutylphthalate, gelatin, polyethylene glycol, preferably PEG 200-600; buffer system, such as lactic acid together with a base such as triethanolamine or sodium hydroxide; waxes, such as beeswax, ozokerite wax, paraffin wax; plant extracts, such as Aloe vera, cornflower, witch hazel, elderflower, cucumber; thickeners; activity enhancers; colourants; perfumes; emulsifiers; and sunscreens.

Cosmetic adjuncts can form up to 50% by weight of the emulsion and can conveniently form the balance of the emulsion.

## Process for preparing the emulsion

The invention also provides a process for the preparation of an emulsion for topical application to skin and/or hair which comprises the step of incorporating into the emulsion a polyol fatty acid polyester and an emulsifier as herein defined.

## Use of the emulsion

The emulsion according to the invention is intended primarily as a product for topical application to human skin, in particular to form an occlusive layer thereon, to reduce moisture loss. The skin can thereby be protected from adverse climatic conditions, for example from excessive exposure to sun and wind, or from detergent damage, for example that following immersion of the hands in aqueous detergent solution when washing dishes or clothes.

The emulsion can also act as a carrier for sunscreen agents, perfumes or germicides, or other skin benefit agents, whose presence on the skin surface can be prolonged due to the presence of the occlusive film of polyol fatty acid polyester.

The emulsion can also be used to treat the hair and the scalp.

In use, a small quantity of the emulsion, for example from 1 to 5 ml, is applied to a selected area of skin or hair, from a suitable container or applicator and, if necessary, it is then spread over and/or rubbed into the skin or hair using the hand or fingers or a suitable spreading device.

## PRODUCT FORM AND PACKAGING

The topical skin and/or hair treatment emulsion of the invention can be formulated as a lotion having a viscosity of from 50 to 10,000 mPa.s, a fluid cream having a viscosity of from 10,000 to 20,000 in mPa.s, or a cream having a viscosity of from 20,000 to 100,000 mPa.s, or above. The emulsion can be packaged in a suitable container to suit its viscosity and intended use by the consumer. For example a lotion or a fluid cream can be packaged in a bottle or a roll-ball applicator or a propellant-driven aerosol device or a container fitted with a pump suitable for finger operation. When the emulsion is a cream, it can simply be stored in a non-deformable bottle or squeeze container, such as a tube or a lidded jar.

The invention accordingly also provides a closed container containing a cosmetically acceptable emulsion as herein defined.

## The Occlusivity Value Test

The preferred polyol fatty acid polyesters according to the invention are characterised as possessing an ability to form an occlusive layer, when the emulsion is applied topically to human skin, so as to reduce the loss of moisture from the skin surface.

In view of the wide variation in the characteristics and properties of human skin, as seen amongst a group of individuals of differing ages, races and habitat, it is necessary to provide a standard in vitro test which is readily reproducable, in order to measure the occlusivity of the polyol fatty acid polyester.

An empirical test has accordingly been devised using a standard viscose cellulose film, namely Visking dialysis tubing available from Medicell International Ltd. as a substitute for human skin. This film has a molecular weight cut-off of from 12,000 to 14,000.

In this test, the occlusivity of a film of polyol fatty acid polyester to the passage of water vapour applied to the dialysis film is measured in a standard manner as follows:

## Preparation of occlusivity cell

A 5ml beaker, for example a Dispo beaker available from American Scientific Products, the diameter of the open end of which is 25mm (i.e. an area of ~5cm$^2$), is used to provide an occlusivity cell.

1ml distilled water is introduced into the beaker and a film of Visking dialysis tubing is stretched across the open end of the beaker and fixed in place with adhesive, for example Assembly Aid Adhesive (3M).

The rate of water loss through the Visking film at 20°C, at atmospheric pressure and at 50% external relative humidity, is determined by measuring the decrease in weight of the beaker with time using a Sartorius 4503 microbalance, with a D to A converter feeding the output to a chart recorder.

After a steady-state water loss rate has been established, a product whose Occlusivity Value is to be tested, i.e. a polyol fatty acid polyester, is applied as a film to the surface of the Visking dialysis tubing. When the test

substance is liquid or a soft solid, it can be applied using a plastic-gloved finger. When the test material is a solid, it is necessary first to melt it as it is applied to the surface of the Visking dialysis film.

The new steady-state water loss rate, under the same physical conditions of pressure, temperature and relative humidity, is then recorded after excess water from the product has been lost.

Occlusivity of the product film (i.e. the polyol fatty acid polyester) is then calculated as:

$$\%\ \text{occlusivity} = 1 - \frac{\text{water loss rate with product}}{\text{water loss rate without product}} \times 100$$

All water loss rates are corrected for the relatively small rate of water loss through the walls of the beaker (if any). This is determined by measuring the water loss from a beaker where the Visking film is replaced with impermeable aluminium foil.

Occlusivity is normally determined 4 times for each sample. For each measurement, the sample loading is determined from the increase in recorded weight immediately after application to the Visking film of the polyol fatty acid polyester. Since the loading is not reproducable precisely, a straight line is fitted to a loading versus occlusivity plot (by linear regression) and the occlusivity at a typical consumer product loading of 10g/sq m is then interpolated. Experience has shown that about 10mg of the product applied to the Visking film is sufficient to provide an occlusive layer; without an occlusive layer, the film will normally transmit about 25g water vapour/$m^2$/hr.

The Occlusivity Value and other physical properties of a series of polyol fatty acid polyesters

The Occlusivity Value as measured by the above Occlusivity Value Test of a series of polyol fatty acid polyesters is given in Table 2 below. Included in this table are data relating to other physical properties of these polyesters.

In each case the polyol moiety was sucrose, the fatty acid ester moiety being derived either from specific fatty acids or mixed (unspecified) fatty acid from natural vegetable oils or fats.

**Table 2 Physical properties of selected sucrose fatty acid polyesters**

| Code No. | Fatty acid or oil source | Occlusivity value (%) (± 2 SE) @10 $g/m^2$ loading | % solids at 30°C | Slip melting point (°C) | T value [50% solids] (°C) | Hydroxyl value |
|---|---|---|---|---|---|---|
| 1 | palm/palmkernel | 86.8 (6.2) | 35 | 36 | 28 | 2.0 |
| 2 | lauric acid | 89.9 (2.3) | 95 | 39 | 36 | 9.6 |
| 3 | soyabean | 93.2 (2.0) | 65 | 50 | 35 | 8.5 |
| 4 | palm/palmkernel | 89.8 (5.1) | 95 | 45 | 36 | 3.8 |
| 5 | soyabean/palm | 86.3 (5.1) | 60 | 43 | 33 | - |

| Code No. | Fatty acid or oil source | Occlusivity value (%) (± 2 SE) @ 10g/$m^2$ loading | % solids at 30°C | Slip melting point (°C) | T value [50% solids] (°C) | Hydroxyl value |
|---|---|---|---|---|---|---|
| 6 | soyabean | 90.9 (3.6) | 65 | 52 | 38 | 3.3 |
| 7 | soyabean | 94.9 (0.5) | 60 | 45 | 32 | 3.8 |
| 8 | palm/palmkernel | 91.5 (2.5) | 70 | 41 | 33 | 1.3 |
| 9 | soyabean | 89.3 (6.2) | 44 | 42 | 28 | 2.6 |
| 10 | palm | 89.9 (1.6) | 14 | 33 | 19 | 5.5 |
| 11 | soyabean | 79.3 (6.8) | 4 | 30 | 16 | 10.5 |

| Code No. | Fatty acid or oil source | Occlusivity value (%) (± 2 SE) @ $10 g/m^2$ loading | % solids at 30°C | Slip melting point (°C) | T value [50% solids] (°C) | Hydroxyl value |
|---|---|---|---|---|---|---|
| 12. | soyabean | 11.8 (5.5) | 0 | 10 | <0 | 7.3 |
| 13. | coconut | 47.2 (13.3) | 0 | 24 | 18 | 7.5 |
| 14. | palm | 63.0 (4.5) | 86 | 49 | 40 | 2.4 |
| 15. | fish | 51.2 (4.6) | 98 | 25 | 11 | - |

Explanation of physical properties

Hydroxyl value is the number of mg of potassium hydroxide required to neutralise the amount of acetic acid capable of combining by acetylation with 1g of the sucrose polyester and accordingly is a measure of the proportion of free hydroxyl groups in the sucrose polyester which have not been esterified with a fatty acid.

Occlusivity Value is a measure of the Occlusivity of the sucrose polyester as obtained by the Occlusivity Value Test as defined and described herein.

T value is that temperature at which N = 50, where 50% of the sucrose fatty acid polyester is solid.

Slip melting point is that temperature at which N = 5, i.e. where 5% of the sucrose fatty acid polyester is solid.

% solids at 30°C is the percentage of the sucrose fatty acid polyester that is solid at 30°C.

Note that the melting characteristics of the sucrose fatty acid polyester, as expressed in terms of T value, slip melting point and % solids at 30°C, are obtained using nuclear magnetic resonance (NMR) measurements. Thus, the N-line is the plot of $N_t$ - values versus the temperature t. The $N_t$-value is conveniently measured by a nuclear magnetic relaxation technique and is a direct measurement of the level of solid fat content at temperature t. This method is described in Fette, Seifen Anstrichmittel 80 (5), 180-186 (1978). To some extent, the measurement of $N_t$ - values is dependent on the temperature profile used to prepare the samples for the NMR measurement. For the purpose of the invention the following preparatory temperature profile is adopted: first 30 minutes at 60°C, followed by 90 minutes at 0°C, 40 hours at 20°C, again 90 minutes at 0°C and finally 60 minutes at the temperature of the measurement, after which the NMR measurement is carried out.

EXAMPLES

The invention is illustrated with reference to the following example formulations in accordance with the invention. Each polyester is identified by a number in parenthesis which refers to the corresponding Code No. in Table 2, where Occlusivity Values and other properties are given.

Examples 1 to 4

These examples illustrate Hand and Body Creams.

| | % w/w | | | |
|---|---|---|---|---|
| Ingredient | 1 | 2 | 3 | 4 |
| Sucrose polyester (1) | 5 | 10 | 15 | 20 |
| Mineral oil | 15 | 10 | 5 | - |
| Stearic acid | 3 | 3 | 3 | 3 |
| Cetostearyl alcohol | 4 | 4 | 4 | 4 |
| Triethanolamine | 1.5 | 1.5 | 1.5 | 1.5 |
| Glycerol | 5 | 5 | 5 | 5 |
| Antioxidant, preservative and perfume | 0.6 | 0.6 | 0.6 | 0.6 |
| water | to 100 | 100 | 100 | 100 |

Examples 5 to 8

These examples also illustrate Hand and Body Creams.

| Ingredient | % w/w 5 | 6 | 7 | 8 |
|---|---|---|---|---|
| Sucrose polyester (3) | 5 | 10 | 15 | 20 |
| Mineral oil | 15 | 10 | 5 | - |
| Stearic acid | 3 | 3 | 3 | 3 |
| Cetostearyl alcohol | 4 | 4 | 4 | 4 |
| Triethanolamine | 1.5 | 1.5 | 1.5 | 1.5 |
| Glycerol | 5 | 5 | 5 | 5 |
| Antioxidant, preservative and perfume | 0.6 | 0.6 | 0.6 | 0.6 |
| water | to 100 | 100 | 100 | 100 |

Examples 9 to 12

These examples illustrate Hand and Body Creams.

| Ingredient | % w/w 9 | 10 | 11 | 12 |
|---|---|---|---|---|
| Sucrose polyester (4) | 5 | 10 | 15 | 20 |
| Mineral oil | 15 | 10 | 5 | - |
| Stearic acid | 3 | 3 | 3 | 3 |
| Cetostearyl alcohol | 4 | 4 | 4 | 4 |
| Triethanolamine | 1.5 | 1.5 | 1.5 | 1.5 |
| Glycerol | 5 | 5 | 5 | 5 |
| Antioxidant, preservative and perfume | 0.6 | 0.6 | 0.6 | 0.6 |
| water | to 100 | 100 | 100 | 100 |

Examples 13 to 16

These examples also illustrate Hand and Body Creams.

| Ingredient | % w/w 13 | 14 | 15 | 16 |
|---|---|---|---|---|
| Sucrose polyester (2) | 5 | 10 | 15 | 20 |
| Mineral oil | 15 | 10 | 5 | - |
| Stearic acid | 3 | 3 | 3 | 3 |
| Cetostearyl alcohol | 4 | 4 | 4 | 4 |
| Triethanolamine | 1.5 | 1.5 | 1.5 | 1.5 |
| Glycerol | 5 | 5 | 5 | 5 |
| Antioxidant, preservative and perfume | 0.6 | 0.6 | 0.6 | 0.6 |
| water | to 100 | 100 | 100 | 100 |

Examples 17 to 20

These examples illustrate hand and body creams with oil continuous phase.

| Ingredient | % w/w 17 | 18 | 19 | 20 |
|---|---|---|---|---|
| Sucrose polyester(10) | 5 | 10 | 15 | 20 |
| Mineral oil | 15 | 10 | 5 | - |
| Ozokerite wax | 8 | 8 | 8 | 8 |
| Glycerol | 10 | 10 | 10 | 10 |
| Arlacel 83 | 1 | 1 | 1 | 1 |
| Magnesium sulphate | 0.2 | 0.2 | 0.2 | 0.2 |
| Preservative, antioxidant | 0.6 | 0.6 | 0.6 | 0.6 |
| Perfume | | | | |
| Water | to 100 | 100 | 100 | 100 |

Examples 21 to 24

These examples illustrate face creams with water continuous phase:

| Ingredient | % w/w 21 | 22 | 23 | 24 |
|---|---|---|---|---|
| Sucrose polyester (9) | 5 | 10 | 15 | 20 |
| Mineral oil | 8 | 8 | 8 | 5 |
| Ceresin wax | 3 | - | - | - |
| Glyceryl monostearate | 2 | 2 | 2 | 2 |
| Cetyl alcohol | 1.5 | 1.5 | 1.5 | 1.5 |
| Brij 35 | 0.5 | 0.5 | 0.5 | 0.5 |
| Span 80 | 1.5 | 1.5 | 1.5 | 1.5 |
| Stearic acid | 2.5 | 2.5 | 2.5 | 2.5 |
| Triethanolamine | 0.7 | 0.7 | 0.7 | 0.7 |
| Carbopol 934* | 0.2 | 0.2 | 0.2 | 0.2 |
| Isopropyl palmitate | 5 | 2 | 2 | - |
| Glycerol | 5 | 5 | - | - |
| Antioxidant, preservative | | | | |
| Perfume | 1 | 1 | 1 | 1 |
| Water | to 100 | 100 | 100 | 100 |

* Carbopol 934 is Carbomer 934 (CTFA)

Examples 25 to 28

These examples illustrate hand and body creams with oil continuous phase.

| Ingredient | % w/w 25 | 26 | 27 | 28 |
|---|---|---|---|---|
| Sucrose polyester (1) | 2 | 5 | 8 | 10 |
| Sucrose polyester (9) | 3 | 5 | 7 | 10 |
| Mineral oil | 15 | 10 | 5 | - |
| Ozokerite wax | 8 | 8 | 8 | 8 |
| Glycerol | 10 | 10 | 10 | 10 |
| Arlacel 83 | 1 | 1 | 1 | 1 |
| Magnesium sulphate | 0.2 | 0.2 | 0.2 | 0.2 |
| Preservative, antioxidant perfume | 0.6 | 0.6 | 0.6 | 0.6 |
| Water | to 100 | 100 | 100 | 100 |

Examples 29 to 32

These examples illustrate face creams with water continuous phase:

| Ingredient | % w/w 29 | 30 | 31 | 32 |
|---|---|---|---|---|
| Sucrose polyester (3) | 2 | 5 | 8 | 10 |
| Sucrose polyester (10) | 3 | 5 | 7 | 10 |
| Mineral oil | 8 | 8 | 8 | 5 |
| Ceresin wax | 3 | - | - | - |
| Glyceryl monostearate | 2 | 2 | 2 | 2 |
| Cetyl alcohol | 1.5 | 1.5 | 1.5 | 1.5 |
| Brij 35 | 0.5 | 0.5 | 0.5 | 0.5 |
| Span 80 | 1.5 | 1.5 | 1.5 | 1.5 |
| Stearic acid | 2.5 | 2.5 | 2.5 | 2.5 |
| Triethanolamine | 0.7 | 0.7 | 0.7 | 0.7 |
| Carbopol 934 | 0.2 | 0.2 | 0.2 | 0.2 |
| Isopropyl palmitate | 5 | 2 | 2 | - |
| Glycerol | 5 | 5 | - | - |
| Antioxidant, preservative | | | | |
| Perfume | 1 | 1 | 1 | 1 |
| Water | to 100 | 100 | 100 | 100 |

Examples 33 to 36

These examples illustrate hand and body creams with oil continuous phase.

| Ingredient | % w/w 25 | 26 | 27 | 28 |
|---|---|---|---|---|
| Sucrose polyester (12) | 5 | - | - | - |
| Sucrose polyester (13) | - | 5 | - | - |
| Sucrose polyester (14) | - | - | 5 | - |
| Sucrose polyester (15) | - | - | - | 5 |
| Mineral oil | 15 | 10 | 5 | - |
| Ozokerite wax | 8 | 8 | 8 | 8 |
| Glycerol | 10 | 10 | 10 | 10 |
| Arlacel 83 | 1 | 1 | 1 | 1 |
| Magnesium sulphate | 0.2 | 0.2 | 0.2 | 0.2 |
| Preservative, antioxidant perfume | 0.6 | 0.6 | 0.6 | 0.6 |
| Water | to 100 | 100 | 100 | 100 |

## Claims

1. Use for the provision of an occlusive layer on skin or hair following topical application thereto of a cosmetic emulsion which comprises in addition to water:
   i. from 1 to 95% by weight of a polyol fatty acid polyester, the polyol having at least 4 free hydroxyl groups, at least 60% of which are then esterified with one or more fatty acids having from 8 to 22 carbon atoms;
   ii. from 1 to 50% by weight of an emulsifier;

   the emulsion having an aqueous phase which forms from 4 to 97% by volume, and a non-aqueous phase which forms from 3 to 96% by volume;
   provided that when the emulsion is an oil-in-water emulsion, the emulsion is substantially free from an emulsifier having an HLB value of <6.

2. Use according to claim 1, in which the polyol fatty acid polyester has an Occlusivity Value of at least 50% as measured by the Occlusivity Value Test.

3. Use according to claim 1 or 2, in which the polyol fatty acid polyester has an Occlusivity Value of at least 70% as measured by the Occlusivity Value Test.

4. Use according to claim 1 or 2, in which the polyol fatty acid polyester has an Occlusivity Value of at least 80% as measured by the Occlusivity Value Test.

5. Use according to any preceding claim, in which the polyol fatty acid polyester is derived from a sugar polyol.

6. Use according to claim 5, in which the sugar polyol is chosen from monosaccharides, disaccharides, trisaccharides and mixtures thereof.

7. Use according to claim 6, in which the disaccharide is sucrose.

8. Use according to claim 5, in which the polyol fatty acid polyester is derived from a sugar alcohol.

9. Use according to any preceding claim, in which the polyol fatty acid polyester is one in which at least 70%

of its free hydroxyl groups of the polyol moiety are esterified.

10. Use according to any preceding claim, in which the fatty acid moiety of the polyol fatty acid polyester is saturated.

11. Use according to any of claims 1 to 9, in which the fatty acid moiety of the polyol fatty acid polyester is unsaturated.

12. Use according to any preceding claim, in which the fatty acid moiety of the polyol fatty acid polyester is a branched chain fatty acid.

13. Use according to any preceding claim, in which the fatty acid moiety of the polyol fatty acid polyester is derived from one or more oils or one or more fats.

14. Use according to any preceding claim, in which the emulsifier has an average HLB value of from 1 to 6.

15. Use according to any preceding claim, which is a water-in-oil emulsion.

16. Use according to any of claims 1 to 13, in which the emulsifier has an average HLB value of from 6 to 14.

17. Use according to claim 16, which is an oil-in-water emulsion.

**Patentansprüche**

1. Verwendung einer kosmetischen Emulsion zur Bereitstellung einer Okklusivschicht auf Haut oder Haar nach topischer Aufbringung darauf, die zusätzlich zu Wasser umfaßt:
   (i) 1 bis 95 Gew.-% eines Polyolfettsäurepolyesters, wobei der Polyol mindestens vier freie Hydroxylgruppen besitzt, von denen dann wenigstens 60 % mit einer oder mehreren Fettsäuren mit 8 bis 22 Kohlenstoffatomen verestert sind;
   ii. 1 bis 50 Gew.-% eines Emulgators;
   wobei die Emulsion eine wäßrige Phase, die 4 bis 97 Vol.-% ausmacht, und eine nichtwäßrige Phase, die 3 bis 96 Vol.-% ausmacht, besitzt;
   mit der Maßgabe, daß, wenn die Emulsion eine Öl-in-Wasser Emulsion ist, die Emulsion im wesentlichen frei von einem Emulgator mit einem HLB-Wert von < 6 ist.

2. Verwendung nach Anspruch 1, wobei der Polyolfettsäurepolyester einen Okklusivitätswert von wenigstens 50% wie durch den Okklusivitätswerttest gemessen besitzt.

3. Verwendung nach Anspruch 1 oder 2, wobei der Polyolfettsäurepolyester einen Okklusivitätswert von wenigstens 70 % wie durch den Okklusivitätswerttest gemessen besitzt.

4. Verwendung nach Anspruch 1 oder 2, wobei der Polyolfettsäurepolyester einen Okklusivitätswert von wenigstens 80 % wie durch den Okklusivitätswerttest gemessen besitzt.

5. Verwendung nach einem vorhergehenden Anspruch, wobei der Polyolfettsäurepolyester von einem Zuckerpolyol abgeleitet ist.

6. Verwendung nach Anspruch 5, wobei der Zuckerpolyol aus Monosacchariden, Disacchariden, Trisacchariden und Mischungen davon ausgewählt wird.

7. Verwendung nach Anspruch 6, wobei das Disaccharid Sucrose ist.

8. Verwendung nach Anspruch 5, wobei der Polyolfettsäurepolyester von einem Zuckeralkohol abgeleitet ist.

9. Verwendung nach einem vorhergehenden Anspruch, wobei der Polyolfettsäurepolyester einer ist, bei dem wenigstens 70 % seiner freien Hydroxylgruppen des Polyolrestes verestert sind.

10. Verwendung nach einem vorhergehenden Anspruch, wobei der Fettsäurerest des Polyolfettpolyesters gesättigt ist.

11. Verwendung nach einem der Ansprüche 1 bis 9, wobei der Fettsäurerest des Polyolfettsäurepolyesters ungesättigt ist.

12. Verwendung nach einem vorhergehenden Anspruch, wobei der Fettsäurerest des Polyolfettsäurepolyesters eine verzweigtkettige Fettsäure ist.

13. Verwendung nach einem vorhergehenden Anspruch, wobei der Fettsäurerest des Polyolfettsäurepolyesters von ein oder mehreren Ölen oder ein oder mehreren Fetten abgeleitet ist.

14. Verwendung nach einem vorhergehenden Anspruch, wobei das Emulgiermittel einen Durchschnitts-HLB-Wert von 1 bis 6 besitzt.

15. Verwendung nach einem vorhergehenden Anspruch, wobei eine Wasser-in-Öl-Emulsion vorliegt.

16. Verwendung nach einem der Anspruch 1 bis 13, wobei das Emulgiermittel einen Durchschnitts-HLB-Wert von 6 bis 14 besitzt.

17. Verwendung nach Anspruch 16, wobei eine Öl-in-Wasser-Emulsion vorliegt.

## Revendications

1. Utilisation, pour fournir une couche occlusive sur la peau ou les cheveux suite à l'application topique sur celle-ci, d'une émulsion cosmétique comprenant, outre l'eau :
   i. de 1 à 95% en masse d'un polyester d'acide gras et de polyol, le polyol ayant au moins 4 groupes hydroxyles libres, dont au moins 60% sont ensuite estérifiés avec un ou plusieurs acides gras ayant de 8 à 22 atomes de carbone ;
   ii. de 1 à 50% en masse d'un émulsifiant ;

   l'émulsion ayant une phase aqueuse qui représente de 4 à 97% en volume, et une phase qui représente de 3 à 96% en volume ;
   à condition que lorsque l'émulsion est une émulsion huile dans l'eau, l'émulsion ne contienne substantiellement pas d'émulsifiant ayant une valeur HLB < 6.

2. L'utilisation selon la revendication 1, dans laquelle le polyester d'acide gras et de polyol a une Valeur de la Capacité d'Occlusion d'au moins 50%, telle que mesurée par le Test de la Valeur de la Capacité d'Occlusion.

3. L'utilisation selon la revendication 1 ou 2, dans laquelle le polyester d'acide gras et de polyol a une Valeur de la Capacité d'Occlusion d'au moins 70% telle que mesurée par le Test de la Valeur de la Capacité d'Occlusion.

4. L'utilisation selon la revendication 1 ou 2, dans laquelle le polyester d'acide gras et de polyol a une Valeur de la Capacité d'Occlusion d'au moins 80% telle que mesurée par le Test de la Valeur de la Capacité d'Occlusion.

5. L'utilisation selon l'une des revendications précédentes, dans laquelle le polyester d'acide gras et de polyol est dérivé d'un polyol de sucre.

6. L'utilisation selon la revendication 5, dans laquelle le polyol de sucre est choisi à partir des monosaccharides, des disaccharides, des trisaccharides et des mélanges de ceux-ci.

7. L'utilisation selon la revendication 6, dans laquelle le disaccharide est du saccharose.

8. L'utilisation selon la revendication 5, dans laquelle le polyester d'acide gras et de polyol est dérivé d'un alcool de sucre.

9. l'utilisation selon l'une des revendications précédentes, dans laquelle le polyester d'acide gras et de polyol est un polyester dans lequel au moins 70% des groupes hydroxyles libres de la partie de molécule de

polyol sont estérifiés.

10. L'utilisation selon l'une des revendications précédentes, dans laquelle la partie de molécule d'acide gras du polyester d'acide gras et de polyol est saturée.

11. L'utilisation selon l'une des revendications 1 à 9, dans laquelle la partie de molécule d'acide gras du polyester d'acide gras et de polyol est insaturée.

12. L'utilisation selon l'une des revendications précédentes, dans laquelle la partie de molécule d'acide gras du polyester d'acide gras et de polyol est un acide gras à chaîne ramifiée.

13. L'utilisation selon l'une des revendications précédentes, dans laquelle la partie de molécule d'acide gras du polyester d'acide gras et de polyol est dérivée d'une ou plusieurs huiles ou d'une ou plusieurs matières grasses.

14. L'utilisation selon l'une des revendications précédentes, dans laquelle l'émulsifiant a une valeur HLB moyenne allant de 1 à 6.

15. L'utilisation selon l'une des revendications précédentes, qui est une émulsion eau dans l'huile.

16. L'utilisation selon l'une des revendications 1 à 13, dans laquelle l'émulsifiant a une valeur HLB moyenne allant de 6 à 4.

17. L'utilisation selon la revendication 16, qui est une émulsion huile dans l'eau.